# EUROPÄISCHE PATENTSCHRIFT

(11) **EP 0 845 456 B1**
(45) Veröffentlichungstag und Bekanntmachung des Hinweises auf die Patenterteilung: **13.06.2001**
(21) Anmeldenummer: 97119147.3
(22) Anmeldetag: 03.11.1997
(51) Int. Cl.: C07C 227/42

(54) **Verfahren zur Herstellung eines kristallinen Feststoffes aus Glycin-N,N-diessigsäure-Derivaten mit hinreichend geringer Hygroskopizität**
Process for the preparation of a crystalline solid of derivatives of (N,N-diacetic acid) glycine with an adequately reduced hygroscopicity
Procédé de préparation d'un solide cristallin de dérivés de glycine N,N-diacétique possédant une hygroscopie suffisamment réduite

(30) Priorität: 29.11.1996 DE 19649681
(43) Veröffentlichungstag der Anmeldung: 03.06.1998
(73) Patentinhaber: BASF AKTIENGESELLSCHAFT, 67056 Ludwigshafen (DE)
(72) Erfinder: Schönherr, Michael, Dr., 67227 Frankenthal (DE); Rauls, Matthias, Dr., 67117 Limburgerhof (DE); Ascherl, Hermann, 67246 Dirmstein (DE); Letzelter, Thomas, 76855 Annweiler (DE); Baumann, Dieter, 67227 Frankenthal (DE); Potthoff-Karl, Birgit, Dr., 67061 Ludwigshafen (DE)

(56) Entgegenhaltungen:
- WO-A-94/29421
- GB-A- 2 024 203
- US-A- 3 956 379

## Beschreibung

Komplexbildner für Erdalkali- und Schwermetallionen, wie sie beispielsweise in Wasch- und Reinigungsmitteln eingesetzt werden, werden üblicherweise in wäßriger Lösung synthetisiert. Für bestimmte Anwendungsfälle werden sie in fester Form benötigt.

Übliche Verfahren zur Herstellung von Feststoffen aus Lösungen sind insbesondere Kristallisations- und Sprühtrocknungsverfahren. Bekannt ist, daß kristalliner Feststoff, wie er beispielsweise bei Verdampfungs- oder Kühlkristallisationsverfahren anfällt, Kristallwasser enthalten kann und unter Umgebungsbedingungen meist weniger hyroskopisch und lagerstabiler als amorpher Feststoff ist. Durch Sprühtrocknungsverfahren (z.B. im Sprühturm oder im Sprühwirbelbett) dagegen wird der Feststoff amorph erhalten. In dieser Form ist der Feststoff oft stark hygroskopisch und bei offener Lagerung unter Umgebungsbedingungen verliert er innerhalb kurzer Zeit die Rieselfähigkeit. Deshalb werden in der Literatur Maßnahmen beschrieben, um die Lagerstabilität von Sprühpulver zu erhöhen, z.B. die Kompaktierung oder Nachbehandlung von Buildern für Waschmittel mit Benzoesäure in der US-A 3 932 316.

Glycin-N,N-diessigsäure-Derivate als Komplexbildner für Erdalkali- und Schwermetallionen in verschiedensten technischen Anwendungsgebieten sind aus der WO-A 94/29421 bekannt. Diese Glycin-N,N-diessigsäure-Derivate, z.B. α-Alanin-N,N-diessigsäure in Form des Trinatriumsalzes, kristallieren stark gehemmt, so daß übliche Kristallisationsverfahren nicht möglich oder nicht wirtschaftlich sind. Die Nachbehandlung von amorphem Sprühpulver dieser Verbindungen mit Additiven, z.B. Benzoesäure gemäß der US-A 3 932 316, ist für einige Anwendungen nicht erwünscht und kann auch nur in begrenztem Umfang die Lagerstabilität verbessern. Die Stabilität eines kristallinen Feststoffes wird nicht erreicht.

Die US-A 3 956 379 offenbart ein Verfahren zur Herstellung von festem Glycin-N,N-diessigsäure-Natriumsalz-Monohydrat mit verbesserter Hygroskopizität durch Kontakt einer hochkonzentrierten wäßrigen Lösung dieser Verbindung mit einem heißen Gas. Aufgabe der vorliegenden Erfindung war es daher, einen praktisch nichthygroskopischen, stabilen, kristallinen Feststoff aus im wesentlichen Glycin-N,N-diessigsäure-Derivaten bereitzustellen, der weitgehend frei von Additiven ist.

Demgemäß wurde ein Verfahren zur Herstellung eines rieselfähigen und lagerstabilen kristallinen Feststoffes, d. h. der für eine Verarbeitung und Anwendung eine hinreichend geringe Hygroskopizität aufweist, welcher im wesentlichen aus Glycin-N,N-diessigsäure-Derivaten der allgemeinen Formel I 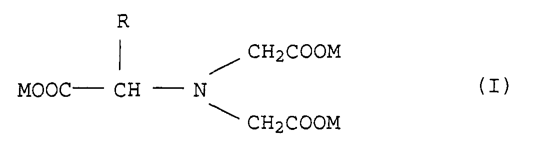 in der
- R: für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel

―(CH₂)ₖ―O―(A¹O)ₘ―(A²O)ₙ―Y

in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 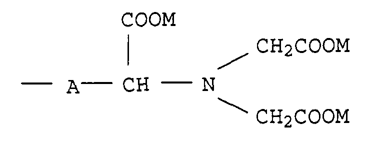 steht, wobei A eine C₁ - bis C₁₂-Alkylen-Brücke oder eine chemische Bindung bezeichnet, und
- M: Wasserstoff, Alkalimetall, Erdalkalimetall, Ammonium oder substituiertes Ammonium in den entsprechenden stöchiometrischen Mengen bedeutet,
besteht, welches dadurch gekennzeichnet ist, daß man den Wassergehalt der die Glycin-N,N-diessigsäure-Derivate I enthaltenden Ausgangsmasse auf einen Wert von 10 bis 30 Gew.-%, bezogen auf die Ausgangsmasse, einstellt und anschließend eine Kristallisation herbeiführt.

Als nichthygroskopisch bzw. hinreichend gering hygroskopisch wird hier ein Feststoff bezeichnet, der bei der offenen Lagerung unter normalen Umgebungsbedingungen, z.B. 25°C und einer relativen Luftfeuchte von 76 %, über einen Zeitraum von mindestens einer Woche seine Konsistenz als rieselfähiges Pulver oder Granulat bewahrt.

Der erfindungsgemäß hergestellte kristalline Feststoff besteht im wesentlichen aus den Verbindungen I, d.h. geringe Mengen von Ausgangs- und/oder Nebenprodukten aus der Herstellung der Glycin-N,N-diessigsäure-Derivate I können mit zugegen sein. Übliche Reinheiten für die Verbindungen I liegen, je nach angewandtem Syntheseverfahren, bei 70 bis 99,9 Gew.-%, insbesondere bei 80 bis 99,5 Gew.-%, jeweils bezogen auf den Feststoffgehalt.

Der Einstellung des Wassergehaltes der Ausgangsmasse kommt eine besondere Bedeutung für die erfindungsgemäße Herstellung des kristallinen Feststoffes zu. Der Wassergehalt ist so zu wählen, daß sich die Kristallisation, gegebenenfalls unter Einbindung von Kristallwasser, optimal ausbilden kann. Er liegt vorzugsweise bei 15 bis 25 Gew.-%, insbesondere bei 18 bis 22 Gew.-%, wobei dieser Wert durch Karl-Fischer-Titration bestimmt wird.

In einer bevorzugten Ausführungsform wird die zur Kristallisation verwendete Ausgangsmasse dadurch erzeugt, daß man eine entsprechende wäßrige Lösung der Glycin-N,N-diessigsäure-Derivate I durch Abdestillation von Wasser ("Eindampfung") auf den erforderlichen Wassergehalt einstellt.

In einer weiteren bevorzugten Ausführungsform wird die zur Kristallisation verwendete Ausgangsmasse mit dem erforderlichen Wassergehalt durch Mischen einer entsprechenden wäßrigen Lösung der Glycin-N,N-diessigsäure-Derivate I mit entwässerten Glycin-N,N-diessigsäure-Derivaten I im geeigneten Verhältnis erzeugt.

In einer weiteren bevorzugten Ausführungsform wird die zur Kristallisation verwendete Ausgangsmasse mit dem erforderlichen Wassergehalt durch Mischung von entwässerten Glycin-N,N-diessigsäure-Derivaten I mit Wasser im geeigneten Verhältnis erzeugt.

Beim Einsatz von entwässerten Glycin-N,N-diessigsäure-Derivate I sind diese in der Regel durch übliche Sprühtrocknungsverfahren hergestellt worden und weisen meist einen Restwassergehalt von 0,1 bis kleiner 10 Gew.-%, insbesondere von 0,5 bis 5 Gew.-%, auf.

Vorteilhafterweise nimmt man die oben beschriebene Erzeugung der Ausgangsmasse und die sich anschließende Kristallisation in derselben Apparatur vor.

Durch die Kristallisation wird die Ausgangsmasse unter günstigen Bedingungen in kurzer Zeit in einen kristallinen Feststoff umgewandelt, der den gestellten Anforderungen hinsichtlich Hygroskopizität, Rieselfähigkeit und Lagerfähigkeit genügt. Es ist daher von Vorteil, die Kristallisation insbesondere in einem günstigen Temperaturbereich, unter Kristallisationskeim-Zugabe und/oder unter mechanischer Beanspruchung vorzunehmen. Die Kristallisation der Ausgangsmasse erfolgt dann in wenigen Minuten.

Man stellt die Temperatur bei der Kristallisation vorzugsweise auf Werte im Bereich von 10 bis 100°C, insbesondere von 20 bis 90°C, vor allem von 40 bis 80°C, ein.

Die Kristallisation wird vorteilhafterweise durch Zugabe von 0,001 bis 50 Gew.-%, vorzugsweise 0,01 bis 10 Gew.-%, bezogen auf die Menge an in der Ausgangsmasse vorhandenen Glycin-N,N-diessigsäure-Derivaten I, kristallinen Glycin-N,N-diessigsäure-Derivaten I als Kristallisationskeime beschleunigt. Dabei sind oft schon die in der Kristallisationsapparatur vorhandenen Produktbeläge aus vorangegangenen Produktionschargen als Keime ausreichend.

Eine sich günstig auswirkende mechanische Beanspruchung kann insbesondere durch eine geeignete Misch-, Rühr-, Knet- oder Extrusions-Apparatur bewirkt werden. Vorteilhaft ist der Einsatz eines Apparates, welcher zwangsgereinigt wird, z.B. eines Discotherm-Reaktors ("DTB"), damit während des Kristallisationsprozesses keine Beläge aufwachsen.

Die Kristallisation wird üblicherweise ohne Zugabe weiterer Lösungs- oder Verdünnungsmittel durchgeführt. Insbesondere wird der Wassergehalt der Ausgangsmasse während der Kristallisation nicht mehr verändert.

Das erfindungsgemäße Verfahren ist vorzugsweise für solche Verbindungen I geeignet, bei denen R für C₁- bis C₂₀-Alkyl, C₂- bis C₂₀-Alkenyl oder einen Rest der Formel 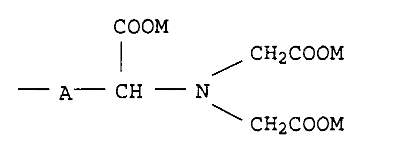 steht.

Besonders bevorzugte werden als Verbindung I α-Alanin-N,N-diessigsäure (R=CH₃) und ihre Alkalimetall-, Ammonium- und substituierten Ammoniumsalze eingesetzt.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Neben Methyl kommen für den Rest R als geradkettige oder verzweigte Alk(en)ylreste vor allem C₂- bis C₁₇-Alkyl und -Alkenyl, hierbei insbesondere geradkettige, von gesättigten oder ungesättigten Fettsäuren abgeleitete Reste, in Betracht. Beispiele für einzelne Reste R sind: Ethyl, n-Propyl, iso-Propyl, n-Butyl, iso-Butyl, sec.-Butyl, tert.-Butyl, n-Pentyl, iso-Pentyl, tert.-Pentyl, Neopentyl, n-Hexyl, n-Heptyl, 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von iso-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als C₁- bis C₁₂-Alkylen-Brücken A dienen vor allem Polymethylengruppierungen der Formel ―(CH₂)ₖ―, worin k eine Zahl von 2 bis 12, insbesondere von 2 bis 8 bezeichnet, d. h. 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte C₁- bis C₁₂-Alkylengruppen auftreten, z. B. ―CH₂CH(CH₃)CH₂―, ―CH₂C(CH₃)₂CH₂―, ―CH₂CH(C₂H₅)― oder ―CH₂CH(CH₃)―.

Die C₁- bis C₃₀-Alkyl- und C₂- bis C₃₀-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierten Alk(en)ylgruppen sind ―CH₂OH, ―CH₂CH₂OH, ―CH₂CH₂-O-CH₃, ―CH₂CH₂―O―CH₂CH₂―O―CH₃, ―CH₂―O―CH₂CH₃, ―CH₂―O―CH₂CH₂―OH, ―CH₂―CHO, ―CH₂―OPh, ―CH₂-COOCH₃ oder ―CH₂CH₂―COOCH₃.

Als Alkoxylat-Gruppierungen kommen insbesondere solche in Betracht, bei denen m und n jeweils für Zahlen von 0 bis 30, vor allem von 0 bis 15 stehen. A¹ und A² bedeuten von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Als fünf- oder sechsgliedrige ungesättigte oder gesättigte heterocyclische Ringe mit bis zu drei Heteroatomen aus der Gruppe Stickstoff, Sauerstoff und Schwefel, welche zusätzlich benzanelliert und durch die bezeichneten Reste substituiert sein können, kommen in Betracht:

Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imidazol, 1,2,3-Triazolidin, 1,2,3- und 1,2,4-Triazol, 1,2,3- , 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-, β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Indol, Isoindolin, Benzoxazol, Indazol, Benzimidazol, Chroman, Isochroman, 2H- und 4H-Chromen, Chinolin, Isochinolin, 1,2,3,4-Tetrahydroisochinolin, Cinnolin, Chinazolin, Chinoxalin, Phthalazin und Benzo-1,2,3-triazin.

N-H-Gruppierungen in den genannten heterocyclischen Ringen sollten möglichst in derivatisierter Form, etwa als N-Alkyl-Gruppierung, vorliegen.

Bei Substitution an den Phenylkernen oder den heterocyclischen Ringen treten vorzugsweise zwei (gleiche oder verschiedene) oder insbesondere ein einzelner Substituent auf.

Beispiele für gegebenenfalls substituierte Phenylalkylgruppen und heterocyclische Ringe tragende Alkylgruppen für R sind Benzyl, 2-Phenylethyl, 3-Phenylpropyl, 4-Phenylbutyl, o-, m- oder p-Hydroxylbenzyl, o-, m- oder p-Carboxylbenzyl, o-, m- oder p-Sulfobenzyl, o-, m- oder p-Methoxy oder -Ethoxycarbonylbenzyl, 2-Furylmethyl, N-Methylpiperidin-4-ylmethyl oder 2-, 3- oder 4-Pyridinylmethyl.

Bei Substitution an Phenylkernen und auch an heterocyclischen Ringen treten bevorzugt wasserlöslich machende Gruppen wie Hydroxylgruppen, Carboxylgruppen oder Sulfogruppen auf.

Als Beispiele für die genannten C₁- bis C₄-, C₁- bis C₁₂- und C₁- bis C₂₀-Alkylgruppen sind auch die entsprechenden oben aufgeführten Reste für R zu verstehen.

Der erfindungsgemäß hergestellte kristalline Feststoff eignet sich in besonderem Maße als Komponente für feste Wasch- und Reinigungsmittelformulierungen, die den erfindungsgemäß hergestellten rieselfähigen und lagerstabilen kristallinen Feststoff aus Glycin-N,N-diessigsäure-Derivaten I als Komplexbildner für Erdalkali- und Schwermetallionen in den hierfür üblichen Mengen neben anderen üblichen Bestandteilen solcher Formulierungen enthalten. Zusammensetzungen und übliche Bestandteile derartiger fester Wasch- und Reinigungsmittelformulierungen sind dem Fachmann bekannt und brauchen daher hier nicht näher erläutert zu werden.

Die nachfolgenden Beispiele sollen die Erfindung näher erläutern. Als Glycin-N,N-diessigsäure-Derivat I wurde jeweils α-Alanin-N,N-diessigsäure-Trinatriumsalz (Methylglycin-N,N-diessigsäure, "MGDA") mit einem Reinheitsgrad von ca. 80 Gew.-%, bezogen auf den Feststoffgehalt, eingesetzt. Wenn nichts anderes angegeben ist, bedeuten "%"-Angaben stets "Gew.-%".

### Beispiel 1

650 g MGDA-Lösung (Wassergehalt 60,7 %) wurden bei 3 bar und 176°C auf 20 % Wassergehalt (bezogen auf die Gesamtmasse) in einem Labor-DTB (Inhalt 0,7 1) eingedampft. Die wasserhaltige Schmelze wurde auf 80°C abgekühlt und erstarrte nach der Zugabe von 20 g MGDA-Keimen nach 15 Minuten. Die Drehzahl der Welle betrug 20 U/min. Es wurden 250 g kristalliner Feststoff aus dem DTB ausgetragen, der unter den Testbedingungen (200 Stunden offene Lagerung bei 25°C und 76 % relative Luftfeuchte) noch rieselfähig und lagerstabil war.

### Beispiel 2

325 g sprühgetrocknetes MGDA-Pulver (Wassergehalt 4,2 %) und 128 g MGDA-Lösung (Wassergehalt 60,7 %) wurden in einem Labor-DTB (Inhalt 0,7 l) vermischt und auf 60°C erhitzt. Die Drehzahl der Welle betrug 20 U/min. Die Mischung erstarrte innerhalb von 10 Minuten. Es wurden 450 g kristalliner Feststoff erhalten, der unter den Testbedingungen (200 Stunden offene Lagerung bei 25°C und 76 % relative Luftfeuchte) noch rieselfähig und lagerstabil war.

### Beispiel 3

a) 14 kg sprühgetrocknetes MGDA-Pulver (Wassergehalt 4 %) und 5,5 kg MGDA-Lösung (Wassergehalt 60 %) wurden in einem gereinigten DTB (Inhalt 30 l) bei 8 U/min vermischt. Der Heizmantel des DTB wurde auf 60°C erwärmt. Die Mischung erstarrte ohne Keimzugabe innerhalb von 15 Minuten. Es wurden 13,6 kg kristalliner Feststoff entleert, der unter den Testbedingungen (200 Stunden offene Lagerung bei 25°C und 76 % relative Luftfeuchte) noch rieselfähig und lagerstabil war.
b) Der Versuch wurde viermal unter gleichen Bedingungen wiederholt, jedoch wurden Pulver und Lösung im nichtgereinigten Apparat vermischt und erwärmt. Die Kristallisation setzte dann schon nach 5 bis 10 Minuten ein. Das Produkt hatte jeweils die gleichen Eigenschaften bzgl. Rieselfähigkeit und Lagerstabilität.

### Vergleichsbeispiele A und B

Zum Vergleich wurde MGDA nach üblichen Methoden sprühgetrocknet (A) und sprühgetrocknet, kompaktiert und mit 1 % Benzoesäure behandelt (B). Produkt A wies unter den Testbedingungen (offene Lagerung bei 25°C und 76 % relative Luftfeuchte) bereits nach 3 Stunden, Produkt B bereits nach 115 Stunden keine ausreichende Rieselfähigkeit und Lagerstabilität mehr auf.

## Patentansprüche

1. Verfahren zur Herstellung eines rieselfähigen und lagerstabilen kristallinen Feststoffes, welcher im wesentlichen aus Glycin-N,N-diessigsäure-Derivaten der allgemeinen Formel I 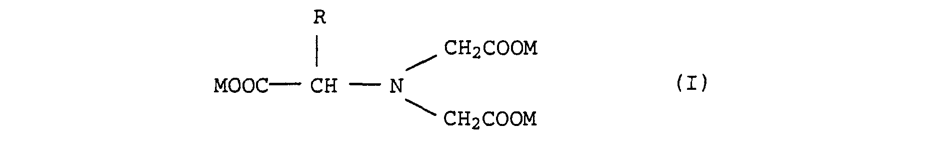 in der
R für C₁- bis C₃₀-Alkyl oder C₂- bis C₃₀-Alkenyl, welche zusätzlich als Substituenten bis zu 5 Hydroxylgruppen, Formylgruppen, C₁- bis C₄-Alkoxygruppen, Phenoxygruppen oder C₁- bis C₄-Alkoxycarbonylgruppen tragen und durch bis zu 5 nicht benachbarte Sauerstoffatome unterbrochen sein können, Alkoxylat-Gruppierungen der Formel
―(CH₂)ₖ―O―(A¹O)ₘ―(A²O)ₙ―Y
in der A¹ und A² unabhängig voneinander 1,2-Alkylengruppen mit 2 bis 4 C-Atomen bezeichnen, Y Wasserstoff, C₁- bis C₁₂-Alkyl, Phenyl oder C₁- bis C₄-Alkoxycarbonyl bedeutet und k für die Zahl 1, 2 oder 3 sowie m und n jeweils für Zahlen von 0 bis 50 stehen, wobei die Summe aus m + n mindestens 4 betragen muß, Phenylalkylgruppen mit 1 bis 20 C-Atomen im Alkyl, einen fünf- oder sechsgliedrigen ungesättigten oder gesättigten heterocyclischen Ring mit bis zu drei Heteroatomen aus der Gruppe Stickstoff, Sauerstoff und Schwefel, welcher zusätzlich benzanelliert sein kann, wobei alle bei den Bedeutungen für R genannten Phenylkerne und heterocyclischen Ringe noch zusätzlich als Substituenten bis zu drei C₁- bis C₄-Alkylgruppen, Hydroxylgruppen, Carboxylgruppen, Sulfogruppen oder C₁- bis C₄-Alkoxycarbonylgruppen tragen können, oder einen Rest der Formel 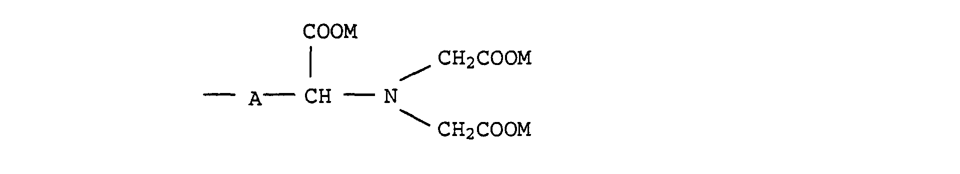 steht, wobei A eine C₁- bis C₁₂-Alkylen-Brücke oder eine chemische Bindung bezeichnet, und
M Wasserstoff, Alkalimetall, Erdalkalimetall, Ammonium oder substituiertes Ammonium in den entsprechenden stöchiometrischen Mengen bedeutet,
besteht, dadurch gekennzeichnet, daß man den Wassergehalt der die Glycin-N,N-diessigsäure-Derivate I enthaltenden Ausgangsmasse auf einen Wert von 10 bis 30 Gew.-%, bezogen auf die Ausgangsmasse, einstellt und anschließend eine Kristallisation herbeiführt.

2. Verfahren nach Anspruch 1, dadurch gekennzeichnet, daß man den Wassergehalt der Ausgangsmasse auf 15 bis 25 Gew.-%, bezogen auf die Ausgangsmasse, einstellt.

3. Verfahren nach Anspruch 1 oder 2, dadurch gekennzeichnet, daß man die zur Kristallisation verwendete Ausgangsmasse dadurch erzeugt, daß man eine entsprechende wäßrige Lösung der Glycin-N,N-diessigsäure-Derivate I durch Abdestillation von Wasser auf den erforderlichen Wassergehalt einstellt.

4. Verfahren nach Anspruch 1 oder 2, dadurch gekennzeichnet, daß man die zur Kristallisation verwendete Ausgangsmasse mit dem erforderlichen Wassergehalt durch Mischen einer entsprechenden wäßrigen Lösung der Glycin-N,N-diessigsäure-Derivate I mit entwässerten Glycin-N,N-diessigsäure-Derivaten I im geeigneten Verhältnis erzeugt.

5. Verfahren nach Anspruch 1 oder 2, dadurch gekennzeichnet, daß man die zur Kristallisation verwendete Ausgangsmasse mit dem erforderlichen Wassergehalt durch Mischen von entwässerten Glycin-N,N-diessigsäure-Derivaten I mit Wasser im geeigneten Verhältnis erzeugt.

6. Verfahren nach den Ansprüchen 1 bis 5, dadurch gekennzeichnet, daß man die Kristallisation in einem Temperaturbereich von 10 bis 100°C vornimmt.

7. Verfahren nach den Ansprüchen 1 bis 6, dadurch gekennzeichnet, daß man die Kristallisation durch Zugabe von 0,001 bis 50 Gew.-%, bezogen auf die Menge an in der Ausgangsmasse vorhandenen Glycin-N,N-diessigsäure-Derivaten I, kristallinen Glycin-N,N-diessigsäure-Derivaten I als Kristallisationskeime beschleunigt.

8. Verfahren nach den Ansprüchen 1 bis 7, dadurch gekennzeichnet, daß man die Kristallisation unter mechanischer Beanspruchung in einer geeigneten Misch-, Rühr-, Knet- oder Extrusions-Apparatur vornimmt.

9. Verfahren nach den Ansprüchen 1 bis 8 zur Herstellung eines rieselfähigen und lagerstabilen kristallinen Feststoffes aus Glycin-N,N-diessigsäure-Derivaten I, bei denen R für C₁- bis C₂₀-Alkyl, C₂- bis C₂₀-Alkenyl oder einen Rest der Formel 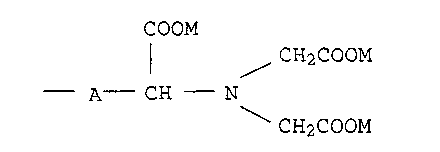 steht.

10. Verfahren nach den Ansprüchen 1 bis 9 zur Herstellung eines rieselfähigen und lagerstabilen kristallinen Feststoffes aus α-Alanin-N,N-diessigsäure oder ihren Alkalimetall-, Ammonium- oder substituierten Aminsalzen.

## Claims

1. A process for preparing a free-flowing crystalline solid which is stable on storage and essentially consists of glycine-N,N-diacetic acid derivatives of the formula I 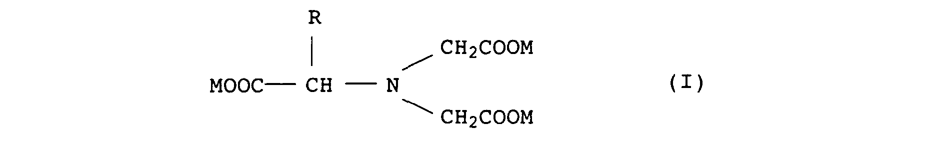 where
R is C₁-C₃₀-alkyl or C₂-C₃₀-alkenyl, each of which may additionally be substituted by up to 5 hydroxyl groups, formyl groups, C₁-C₄-alkoxy groups, phenoxy groups or C₁-C₄-alkoxycarbonyl groups and be interrupted by up to 5 nonadjacent oxygen atoms, alkoxylate groups of the formula
―(CH₂)ₖ―O―(A¹O)ₘ―(A²O)ₙ―Y
where A¹ and A² are, independently of one another, 1,2-alkylene groups having 2 to 4 carbon atoms, Y is hydrogen, C₁-C₁₂-alkyl, phenyl or C₁-C₄-alkoxycarbonyl, and k is 1, 2 or 3, and m and n are each from 0 to 50, where the total of m + n must be at least 4, phenylalkyl groups having 1 to 20 carbon atoms in the alkyl, a five- or six-membered unsaturated or saturated heterocyclic ring which has up to three heteroatoms from the group of nitrogen, oxygen and sulfur and may additionally be benzo-fused, it being possible for all the phenyl nuclei and heterocyclic rings mentioned for the meanings of R additionally to be substituted by up to three C₁-C₄-alkyl groups, hydroxyl groups, carboxyl groups, sulfo groups or C₁-C₄-alkoxycarbonyl groups, or a radical of the formula 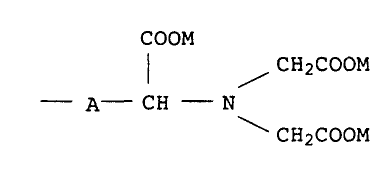 where A is a C₁-C₁₂-alkylene bridge or a chemical bond, and
M is hydrogen, alkali metal, alkaline earth metal, ammonium or substituted ammonium in the appropriate stoichiometric amounts,
which comprises adjusting the water content of the starting material containing the glycine-N,N-diacetic acid derivatives I to a value of from 10 to 30% by weight, based on the starting material, and subsequently bringing about crystallization.

2. A process as claimed in claim 1, wherein the water content of the starting material is adjusted to from 15 to 25% by weight, based on the starting material.

3. A process as claimed in claim 1 or 2, wherein the starting material used for the crystallization is produced by adjusting an appropriate aqueous solution of the glycine-N,N-diacetic acid derivatives I to the required water content by distilling off water.

4. A process as claimed in claim 1 or 2, wherein the starting material with the required water content used for the crystallization is produced by mixing an appropriate aqueous solution of the glycine-N,N-diacetic acid derivatives I with dehydrated glycine-N,N-diacetic acid derivatives I in the suitable ratio.

5. A process as claimed in claim 1 or 2, wherein the starting material with the required water content used for the crystallization is produced by mixing dehydrated glycine-N,N-diacetic acid derivatives I with water in the suitable ratio.

6. A process as claimed in any of claims 1 to 5, wherein the crystallization is carried out at a temperature in the range from 10 to 100°C.

7. A process as claimed in any of claims 1 to 6, wherein the crystallization is speeded up by adding from 0.001 to 50% by weight, based on the amount of glycine-N,N-diacetic acid derivatives I present in the starting material, of crystalline glycine-N,N-diacetic acid derivatives I as crystallization nuclei.

8. A process as claimed in any of claims 1 to 7, wherein the crystallization is carried out under mechanical stress in a suitable mixing, stirring, kneading or extrusion apparatus.

9. A process as claimed in any of claims 1 to 8 for preparing a free-flowing crystalline solid which is stable on storage from glycine-N,N-diacetic acid derivatives I where R is C₁-C₂₀-alkyl, C₂-C₂₀-alkenyl or a radical of the formula

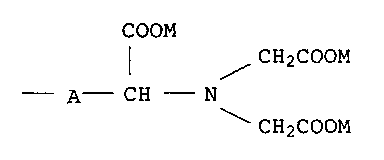

10. A process as claimed in any of claims 1 to 9 for preparing a free-flowing crystalline solid which is stable on storage from α-alanine-N,N-diacetic acid or its alkali metal, ammonium or substituted amine salts.

## Revendications

1. Procédé de préparation d'un solide cristallin fluide et stable à l'entreposage, qui est constitué essentiellement de dérivés de l'acide glycine-N,N-diacétique de formule générale I 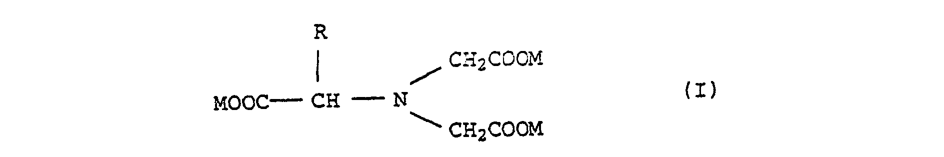 dans laquelle
R représente un groupe alkyle en C₁ à C₃₀, ou alcényle en C₂ à C₃₀, lequel peut porter en plus, en tant que substituants, jusqu'à 5 groupes hydroxyle, groupes formyle, groupes alcoxy en C₁ à C₄, groupes phénoxy ou groupes (alcoxy en C₁ à C₄)-carbonyle et peuvent être interrompus par jusqu'à 5 atomes d'oxygène non vicinaux, des groupes alcoxylates de formule
―(CH₂)ₖ―O―(A¹O)ₘ―(A²O)ₙ―Y
dans laquelle A¹ et A² représentent, indépendamment l'un de l'autre, des groupes 1,2-alkylènes ayant 2 à 4 atomes de C, Y représente un atome d'hydrogène, un groupe alkyle en C₁ à C₁₂, phényle ou alcoxy carbonyle en C₁ à C₄ et k représente le nombre 1, 2 ou 3, et m et n représentent chacun un nombre de 0 à 50, la somme m + n devant valoir au moins 4, des groupes phénylalkyle ayant 1 à 20 atomes de C dans le groupe alkyle, un cycle hétérocyclique, insaturé ou saturé, à 5 ou 6 maillons, ayant jusqu'à 3 hétéro-atomes choisis dans le groupe formé par les atomes d'azote, d'oxygène et de soufre, lequel cycle peut encore être benzocondensé, tous les cycles hétérocycliques et noyaux phényle cités pour les significations de R pouvant encore porter en plus comme substituants jusqu'à 3 groupes alkyle en C₁ à C₄, groupes hydroxyle, groupes carboxyle, groupes sulfo ou groupes (alcoxy en C₁ à C₄)-carbonyle ou bien un résidu de formule 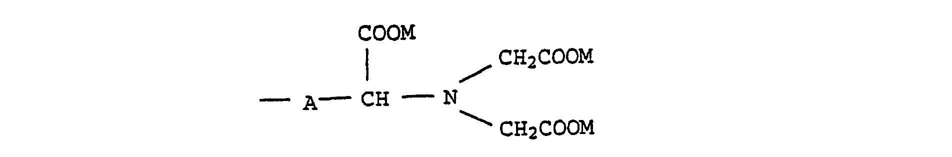 dans laquelle A représente un pont alkylène en C₁ à C₁₂ ou une liaison chimique, et
M représente un atome d'hydrogène, de métal alcalin, de métal alcalino-terreux, un ion ammonium ou un ion ammonium substitué dans les quantités stoechiométriques appropriées,
caractérisé en ce que l'on ajuste la teneur en eau de la masse de départ contenant les dérivés de l'acide glycine-N,N-diacétique I à une valeur de 10 à 30% en poids, par rapport à la masse de départ, puis on réalise une cristallisation.

2. Procédé selon la revendication 1, caractérisé en ce que l'on ajuste la teneur en eau de la masse de départ à 15 à 25% en poids, par rapport à la masse de départ.

3. Procédé selon la revendication 1 ou 2, caractérisé en ce que l'on produit la masse de départ utilisée pour la cristallisation en ajustant une solution aqueuse appropriée des dérivés de l'acide glycine-N,N-diacétique I jusqu'à la teneur en eau requise en éliminant l'eau par distillation.

4. Procédé selon la revendication 1 ou 2, caractérisé en ce que l'on produit la masse de départ utilisée pour la cristallisation avec la teneur en eau requise, en mélangeant une solution aqueuse appropriée des dérivés de l'acide glycine-N,N-diacétique I avec des dérivés déshydratés de l'acide glycine-N,N-diacétique Idans un rapport approprié.

5. Procédé selon la revendication 1 ou 2, caractérisé en ce que l'on produit la masse de départ utilisée pour la cristallisation avec la teneur en eau requise en mélangeant des dérivés déshydratés d'acide glycine-N,N-diacétique I avec de l'eau dans un rapport approprié.

6. Procédé selon les revendications 1 à 5, caractérisé en ce que l'on procède à la cristallisation dans une plage de températures de 10 à 100°C.

7. Procédé selon les revendications 1 à 6, caractérisé en ce que l'on accélère la cristallisation en ajoutant 0,001 à 50% en poids de dérivés de l'acide glycine-N,N-diacétique cristallins I en tant que germes de cristallisation, par rapport à la quantité de dérivés de l'acide glycine-N,N-diacétique présents dans la masse de départ.

8. Procédé selon la revendication 1 à 7, caractérisé en ce que l'on procède à la cristallisation sous une sollicitation mécanique dans un appareil de mélange, d'agitation, de malaxage ou d'extrusion approprié.

9. Procédé selon les revendications 1 à 8, pour préparer un solide cristallin fluide et stable à l'entreposage constitué de dérivés de l'acide glycine-N,N-diacétique , dans lesquels R représente un groupe alkyle en C₁ à C₂₀, alcényle en C₂ à C₂₀, ou un résidu de formule

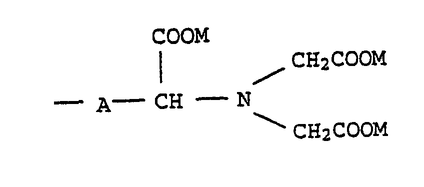

10. Procédé selon les revendications 1 à 9, pour préparer un solide cristallin fluide et stable à l'entreposage constitué d'acide α-alanine-N,N-diacétique ou de ses sels de métal alcalin, d'ammonium ou de sels d'amines substitués.
